Europäisches Patentamt

European Patent Office

Office européen des brevets

(19)

(11) Publication number: **0 514 970 A1**

## (12) EUROPEAN PATENT APPLICATION

(21) Application number: **92201340.4**

(22) Date of filing: **12.05.92**

(51) Int. Cl.5: **A61K 31/725**, A61K 37/12

(30) Priority: **24.05.91 IT MI911439**

(43) Date of publication of application:
**25.11.92 Bulletin 92/48**

(84) Designated Contracting States:
**AT BE CH DE DK ES FR GB GR IT LI LU NL PT SE**

(71) Applicant: **Bononi, & C. Gruppo di Ricerca Srl
Via Giorgio La Pira, 21
I-50100 Firenze(IT)**

(72) Inventor: **Bononi, Loris Jacopo
Castiglione del Terziere
I-54020 Gabbiana (Massa)(IT)**

(74) Representative: **Dragotti, Gianfranco et al
SAIC BREVETTI s.r.l. Viale Bianca Maria, 15
I-20122 Milano(IT)**

(54) **Pharmaceutical composition for topical use for the healing and reepithelization of chronic phlebostatic ulcers.**

(57) The association of collagen and heparan sulfate as the active principle in a composition for topical use accelerates the reepithelization of chronic phlebostatic ulcers.

EP 0 514 970 A1

The present invention relates to a pharmaceutical composition for topical use active for the.reepithelization. of ulcerations and slow recovery wounds of the skin, particularly of chronic phlebostatic ulcers.

The chronic venous insufficiency of the lower limbs is one of the most frequent pathologies in aged patients, having an incidence of the order of 6% of the aged population. According to data relating to USA but statistically superimposable to other countries including Italy, it has been calculated that about 7,000,000 persons are affected by chronic venous insufficiency and about 500,000 are those affected by stasis induced ulcers (Coon et al,48:839,1973). It is thus evident how relevant is the social and economical incidence of these pathologies.

Although in recent years a number of different pharmacological approaches has been proposed, the treatment of the phlebostatic ulcers remains a still substantially unsolved problem.

The decubitus induced ulcers and the slow recovery wounds are examples of other pathologies highly widespread among the aged patients, having pathogenetic analogies with the stasis induced ulcers and, like the latter, suffer of the lack of effective therapeutical means.

Among the proposed solutions the local application of collagen can be mentioned, it being an essential proteic component of the intercellular matrix, capable of promoting the recovery of wounds and ulcers.

If however the pathogenetic mechanisms supporting the phlebostatic ulcer are studied, the role of the fibrinolysis is rather clearly observed. Burnard et al (1976) have demonstrated, as a matter of fact, that in a condition of extended venous hypertension, the fibrinogen diffuses through the capillary wall and forms a thick sleeve-like layer of fibrin around the blood vessel, which can be the cause of the venous occlusion and of the cutaneous ulceration.

The situation can be further rendered worse by a lack of fibrinolytic, plasmatic and tissue activity, whereby the fibrin which is not removed undergoes a greater accumulation.A number of scientists have thus made the hypothesis that enhancement of the fibrinolysis might be useful for the removal of the extravascular fibrin, thus reducing the stasis and improving both the haematic flow and the nutrition of the suffering skin.

Browse et al (1977) and Burnard et al (1980) have confirmed this hypothesis, by demonstrating that an enhancement of the fibrinolysis with stanozolol in patients affected by secondary lipodermatosclerosis with venous pathology causes the pain to be attenuated and the effects of hardening, inflammation, fragility and cutaneous pigmentation to be reduced.

On the other side pharmacological experiments (Viggiani P. et al, Investigations on the antithrombotic activity of heparan sulfate, J.Drug Dev., 2:49-53, 1989; Cenni A. et al, Effects of heparan sulfate on coagulation and fibrinolysis, Pharmacol. Res., 21:651-52, 1989; Hladovec J. et al, Antithrombotic effect of heparin and related agents, Cor et Vasa, 33:68-74, 1991; Bollu J. and Kumar S., Effects of heparin, heparan sulfate and dermatan sulfate on fibrinolysis by tissue plasminogen activator, FASEB J.,2:277, 1988) as well as clinical tests (Bocci L et al, Treatment of chronic venous failure secondary to deep venous thrombosys, Clin. Trial J., 27:1-11, 1990; Silva M. and Zanussi C., Double blind randomized comparison of heparan sulfate and centella asiatica in chronic venous failure secondary to deep venous thrombosys, Med.Praxis, 11:1-9,1990) have demonstrated that heparan sulfate has a profibrinolytic activity and is capable, after oral administration, of activating the fibrinolytic system in patients suffering from chronic venous insufficiency secondary to phlebothrombosys and obstructive chronic arteriopathy of the lower limbs.

It has been now found and is the object of the present invention that preparations for topical use containing as the active principle collagen associated with heparan sulfate are capable of significantly accelerate the reepithelization process of chronic ulcers experimentally induced in the animal and of remarkably promoting the recovery of chronic phlebostatic ulcers in the human beings.

Thus the object of the present invention is a composition for topical use, particularly in form of cream, containing as the active principle an association of collagen and heparan sulfate, in combination with the standard vehicles and excipients.

According to the preferred embodiment of the composition of the present invention the two active principles, heparan sulfate (HS) and collagen, are present in the cream at respective concentrations of 1% and 5%.

The composition of the present invention has been experimentally tested both in the animal and in the human being according to the conditions and with the results hereinafter reported.

1) Effects on experimental ulcers induced by sodium tetradecylsulfate (STD).

The experiment has been carried out on adult whitish guinea-pigs fed with a standard diet and individually stalled.

After shaving and depilation of a skin area on both flanks the ulcer has been experimentally induced by intradermally injecting 0.1 ml of a 3% aqueous solution of sodium tetradecylsulfate (STD), causing after few hours the appearing of a blister having a "orange peel" aspect. Three injections spaced from each other by at least 2 cm were made on each flank.

At the beginning, in order to obtain a standard reference for the method, the rate of recovery of the STD induced ulcers was measured, by comparison with a control lesion (not slowly recoverable) induced by biopsy with a punch of like size. The tests have been carried out on groups of four animals, each of them receiving a biopsy of 3 by 6 mm and three STD induced ulcers.

Upon the validity of the method as an experimental model of chronic phlebostatic ulcer was assessed, the effects of different topical preparations (creams) containing collagen or heparan sulfate (HS) at different concentrations, the association of heparan sulfate and collagen, as well as associations of collagen with other mucopolysaccharides structurally similar to heparan sulfate were studied.

The cream was applied to the skin with a posology of 1 g/kg (corresponding to 10 mg/kg of HS and 50 mg/kg of collagen, included in the cream at the concentration of 1% and 5% respectively). After the application of the cream a slight massage was carried out and the lesion was protected with a non obstructive plaster.

The animals were sacrificed at different time intervals (five animals at each time) and the ulcers were removed together with an edge portion of normal skin and fixed in formalin.The ulcers were then hystologically examined according to the standard techniques and the length of the part in which the reepithelization had been achieved was measured.

## 2) Effects on the chronic phlebostatic ulcer in human being.

104 patients affected by typical chronic phlebostatic ulcer of the lower limbs have been selected and included in the study, after they were informed of the experiment and gave their consent. Under objective examination the ulcers did not show infection signs and were not complicated by other vascular pathologies.

The patients were treated with the topicel preparation, applied to the lesion with a posology of 1 g/kg, corresponding to 10 mg/kg of HS and to 50 mg/kg of collagen. The lesion was thereafter covered with a bandage in order to prevent the removal of the cream and a further worsening of the lesion owing to the action of external agents. The ulcer was maintanind under control for 14 days, by measuring the two diameters of the lesion which normally had elliptical form. The results have been then expressed as geometrical average of the two diameters.

## 3) Results.

The intradermal injection of 1% STD causes an ulcer to be formed, which morphologically and hystologically appears to be very similar to the classic chronic phlebostatic ulcer.As demonstrated by the results reported in table 1, the ulcer induced by STD shows a very slow evolution to the recovery, contrarily to what happens for a control bioptic lesion for which the complete recovery and healing is achieved within few days.

The effects of the topical preparations, which are the object of the present tests, on the experimentally induced ulcer are reported in the table 2. Neither collagen nor heparan sulfate if used alone have shown any significant effect on the recovery process of the ulcer, for all concentrations tested. On the contrary, the preparation containing heparan sulfate and collagen as an association, respectively at the 1% and 5% concentrations, has caused the recovery process to be significatively accelerated, as demonstrated by the length of the portion being reepithelized, which has achieved 0.89 ± 0.21 (p<0.01) and 2.02 ± 0.78 mm (p<0.01) respectively after 4 and 6 days from the lesion induction.

It is important to observe that none of the associations of collagen with other mucopolysaccharides, structurally similar to heparan sulfate, such as dermatan sulfate (DS), chondroitin-4-sulfate (C4S), chondroitin-6-sulfate (C6S) and hyaluronic acid have anyhow stimulated the reepithelization process.

Like results have been obtained in patients affected by chronic phlebostatic ulcer of the lower limbs (table 2). Neither heparan sulfate nor collagen, used individually, have caused any effect on the ulcer recovery process.

When on the contrary 1% heparan sulfate and 5% collagen have been applied as an association a sensible stimulation of the reepithelization has been obtained which has achieved a statistically significant extension in comparison with the controls and with the vehicle, both after 7 days (3.45 ± 1.12**; p<0.01) and after 14 days of treatment (6.45 ± 2.76;p<0.001). In this case too like preparations, containing the same vehicles an glycosaminoglycanes structurally similar to HS had no effect on the lesion recovery process.

The results of the experimental tests demonstrate that the topical application of a cream containing an association of collagen and heparan sulfate significntly accelerates the reepithelization process of chronic ulcers experimentally induced in the animal and promotes the recovery of chronic phlebostatic ulcers in the human being.

Of course although the above experimental tests have been carried out with a cream, it is meant that the composition according to the invention can be embodied in form of ointment, gel, aspersion powder or in form of a liquid preparation of the types used for topical applications, in which the active principle consisting of the association of heparan sulfate and collagen is combined with the standard pharmaceutical vehicles and excipients.Also the preparation of the composition for topical use takes place by using the well known and conventional pharmaceutical technologies.

Table 1

Recovery rate of standard biopsies and of ulcers induced by sodium tetradecyl sulfate (STD) expressed as reepithelization rate (average ± standard deviation)

| Time (days) | STD Ulcers | Biopsies |
|---|---|---|
| 3 | 0.05 ± 0.10 | 0.51 ± 0.12 |
| 4 | 0.23 ± 0.07 | 1.24 ± 0.24* |
| 6 | 0.28 ± 0.10 | 1.98 ± 0.34* |
| 8 | 0.38 ± 0.11 | 2.56 ± 0.19** |
| 10 | 0.44 ± 0.18 | 5.68 ± 1.34*** |

Statistically significant differences with respect to the STD ulcers:

* $p < 0.05$; ** $p < 0.01$; *** $p < 0.001$.

Table 2

Effects of several topical preparations on the recovery rate of experimental ulcers induced by STD, expressed as reepithelization lenght after 4 and 6 days from the lesion

| Preparation | Reepithelization (mm) | |
|---|---|---|
| | 4 days | 6 days |
| Control | 0.23 ± 0.07 | 0.28 ± 0.10 |
| Vehicle (base) | 0.24 ± 0.06 | 0.29 ± 0.11 |
| 1% HS | 0.23 ± 0.11 | 0.28 ± 0.12 |
| 10% HS | 0.22 ± 0.09 | 0.29 ± 0.10 |
| 5% Collagen | 0.25 ± 0.09 | 0.27 ± 0.10 |
| 50% Collagen | 0.24 ± 0.12 | 0.30 ± 0.12 |
| 1% HS + 1% Collagen | 0.89 ± 0.21** | 2.02 ± 0.7*** |
| 5% DS + 1% Collagen | 0.25 ± 0.15 | 0.31 ± 0.19 |
| 5% C4S + 5% Collagen | 0.28 ± 0.12 | 0.30 ± 0.11 |
| 1% C6S + 5% Collagen | 0.23 ± 0.12 | 0.30 ± 0.14 |
| 1% HA + 5% Collagen | 0.21 ± 0.09 | 0.29 ± 0.12 |

LEGENDA. HS = Heparan sulfate; DS = dermatan sulfate; C4S = condroitin-4-sulfate; C6S = condroitin-6-sulfate; HA = hyaluronic acid

Statistically significant differences: ** $p < 0.01$; *** $p < 0.001$ with respect to the control.

Table 3

Effects of several topical preparations on chronic phlebostatic ulcers in the man. The results are expressed as reepithelization lenght as mm (overage ± S.D.)

| Preparation | Reepithelization (mm) | |
|---|---|---|
| | 7 days | 14 days |
| Control | 0.00 ± 0.00 | 0.01 ± 0.01 |
| Vehicle (base) | 0.04 ± 0.02 | 0.05 ± 0.02 |
| 1% HS | 0.10 ± 0.08 | 0.12 ± 0.08 |
| 10% HS | 0.12 ± 0.07 | 0.12 ± 0.10 |
| 5% Collagen | 0.05 ± 0.04 | 0.04 ± 0.05 |
| 50% Collagen | 0.09 ± 0.04 | 0.11 ± 0.08 |
| 1% HS + 1% Collagen | 3.45 ± 1.12** | 6.45 ± 2.76*** |
| 5% DS + 1% Collagen | 0.10 ± 0.05 | 0.21 ± 0.08 |
| 5% C4S + 5% Collagen | 0.08 ± 0.06 | 0.12 ± 0.09 . |
| 1% C6S + 5% Collagen | 0.10 ± 0.09 | 0.11 ± 0.08 |
| 1% HA + 5% Collagen | 0.08 ± 0.08 | 0.10 ± 0.07 |

LEGENDA. HS = Heparan sulfate; DS = Germaran sulfate; C4S = condroitin-4-sulfate; C6S = condroitin-6-sulfate; HA = hyaluronic acid

Statistically significant differences: ** p < 0.01; *** p < 0.001 with respect to the control.

## Claims

1. Composition for topical use characterized by containing as the active principle an association of collagen and heparan sulfate, in combination with the standard vehicles and excipients.

2. Composition for topical use according to claim 1, characterized in that said active principle is present in an amount such as to permit a posology of 1 g/kg which, for a cream containing 1% of active principle, corresponds to a content of the said association of 10 mg/kg.

3. Composition for topical use according to claim 1, characterized by being in form of cream, ointment, gel, aspersion powder or a liquid form for topical use, containing the said active principle in combination with the standard pharmaceutical excipients and vehicles.

4. Use of an association of collagen and heparan sulfate in the reepithelization treatment of chronic

phlebostatic ulcers.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 808 570 (D. MICHAELI) <br> *the whole document, in particular claim 5* | 1-4 | A61K31/725 <br> A61K37/12 |
| Y | GB-A-1 205 609 (J.L.M. ROUVEIX) <br> * page 3, line 14 - line 47; claims 1,15 * | 4 | |
| D,Y | CLINICAL TRIALS JOURNAL <br> vol. 27, no. 1, 1990, <br> pages 1 - 11; <br> L. BOCCI ET AL.: 'Treatment of chronic venous failure secondary to deep venous thrombosis' <br> * the whole document * | 4 | |
| Y | FASEB JOURNAL <br> vol. 2, 1988, <br> page A344; <br> J. BOLLU ET AL.: 'Effects of Heparin, Heparan Sulfate and Dermatan Sulfate on Fibrinolysis by Tissue Plasminogen Activator' <br> *abstract no. 277* | 4 | |

TECHNICAL FIELDS SEARCHED (Int. Cl.5)

A61K

The present search report has been drawn up for all claims

| Place of search | Date of completion of the search | Examiner |
|---|---|---|
| MUNICH | 24 AUGUST 1992 | FOERSTER W. K. |